(19) Europäisches Patentamt

European Patent Office

Office européen des brevets

(11) EP 0 720 473 B1

(12) **EUROPÄISCHE PATENTSCHRIFT**

(45) Veröffentlichungstag und Bekanntmachung des Hinweises auf die Patenterteilung:
**11.11.1998 Patentblatt 1998/46**

(21) Anmeldenummer: **94924303.4**

(22) Anmeldetag: **29.07.1994**

(51) Int. Cl.$^6$: **A61K 9/54**, A61K 31/58

(86) Internationale Anmeldenummer:
**PCT/EP94/02531**

(87) Internationale Veröffentlichungsnummer:
**WO 95/08323 (30.03.1995 Gazette 1995/14)**

(54) **BUDESONID-PELLETS MIT KONTROLLIERTEM FREIGABEPROFIL UND VERFAHREN ZU IHRER HERSTELLUNG**

BUDESONIDE PELLETS WITH A CONTROLLED RELEASE PATTERN AND PROCESS FOR PRODUCING THEM

PELLETS DE BUDESONIDE A LIBERATION CONTROLEE ET LEUR PROCEDE DE PREPARATION

(84) Benannte Vertragsstaaten:
**AT BE CH DE DK ES FR GB GR IE IT LI LU MC NL PT SE**

(30) Priorität: **23.09.1993 DE 4332394**

(43) Veröffentlichungstag der Anmeldung:
**10.07.1996 Patentblatt 1996/28**

(73) Patentinhaber:
**DR. FALK PHARMA GMBH**
**D-79041 Freiburg (DE)**

(72) Erfinder:
• **GRUBER, Peter**
**CH-4103 Bottmingen (CH)**
• **LACH, Hans-Joachim**
**D-79353 Bahlingen (DE)**
• **OTTERBECK, Norbert**
**D-88662 Überlingen (DE)**

(74) Vertreter:
**Keller, Günter, Dr. et al**
**Lederer, Keller & Riederer**
**Patentanwälte**
**Prinzregentenstrasse 16**
**80538 München (DE)**

(56) Entgegenhaltungen:
EP-A- 0 145 558       EP-A- 0 453 001
EP-A- 0 484 186       EP-A- 0 485 840
WO-A-91/07172         FR-A- 2 453 640

**Beschreibung**

Die Erfindung betrifft Budesonid-Pellets mit kontrolliertem Freigabeprofil und ein Verfahren zu ihrer Herstellung. Insbesondere betrifft sie Budesonid-Pellets mit einer freigabesteuernden Membran aus zwei separaten Lackschichten.

Budesonid ($16\alpha$, 17-Butylidendioxy-$1\beta$,21-dihydroxy-1,4-pregnadien-3,20-dion) ist ein hochaktives Corticosteroid, das sich als außerordentlich wirksam zur Behandlung inflammatorischer Prozesse des mittleren und tieferen Intestinaltraktes gezeigt hat. So bewirkte Budesonid in oraler Slow-release-Form eine Remission der aktiven Morbus Crohn und besitzt geringe Nebenwirkungen auf die Nebennierenrindenfunktion (vgl. die Pilotstudie in "Der Kassenarzt, 13, Seiten 34 bis 37, 1993). Eine Übersicht über die pharmakologischen Eigenschaften und die therapeutische Wirksamkeit von Budesonid bei Asthma und Rhinitis ist in "Drugs 44(3), 375-407, 1992" angegeben. Budesonid wird wenig resorbiert und unterliegt einem starken first-pass-Effekt. Da entzündliche Prozesse oft größere Abschnitte des Intestinaltraktes befallen, ist eine Arzneiform gefragt, die sich reproduzierbar über weite Bereiche des Darms ausbreitet und dabei erst dort den Wirkstoff freisetzt.

Die WO-A-91/07172 offenbart Pellets, bestehend aus einem Kern, einer Wirkstoffschicht und einer Lackschicht aus Mischungen aus pH-abhängigen Polymeren. Diese Pellets unterscheiden sich hinsichtlich ihrer Freigabecharakteristik von den vorliegend beanspruchten Budesonid-Pellets.

Die EP-A-0 145 558 betrifft galenische Formen zur oralen Anwendung von Sulpirid. Diese Formulierungen weisen kein verzögertes S-förmiges Freigabeprofil auf, sondern es wird eine sehr schnelle Freisetzung im sauren pH-Bereich angestrebt.

Die EP-A-0 453 001 betrifft pharmazeutische Zubereitungen mit verzögerter Freisetzung. Ein S-förmiges Freigabeprofil wird nicht offenbart.

Die EP-A-0 484 186 betrifft Formulierungen und deren Verwendung für die Behandlung von neurologischen Erkrankungen. Es handelt sich um Retardformulierungen, wobei das vorliegende Freigabeprofil nicht offenbart wird.

Aufgabe der Erfindung ist daher die Bereitstellung eines Arzneimittels, das eine optimale Verteilung der geringen Wirkstoffmenge (1 bis 3 mg/Dosis) am Entzündungsort gewährleistet. Ferner soll der Wirkstoff weder im Magen noch im Duodenum bzw. dem proximalen Jejunum freigesetzt werden, sondern ab dem mittleren Jejunum. Ab diesem Abschnitt soll die Freisetzung relativ schnell (ca. 80 bis 90% in 2 Stunden) sein und sich deutlich von einer Retardform (z.B. 90% Freigabe in 6 bis 8 Stunden) unterscheiden.

Weiterhin soll ein reproduzierbarer Wirkungseintritt durch rasche Passage des Arzneimittels durch den Magen gewährleistet werden.

Nach Ablösen einer freigabesteuernden Hülle soll sich der schwerlösliche, sehr fein mikronisierte Wirkstoff rasch im Darmsaft resuspendieren und an der entzündeten Mucosa seine Wirkung entfalten. Weiterhin soll ein Verfahren zur Herstellung der Pellets zur Verfügung gestellt werden.

Diese Aufgabe wird am besten von Pellets, abgefüllt z.B. in Hartgelatinekapseln, gelöst. Nach dem Zerfall der Kapsel im Magen werden sie entsprechend der im Magen gerade herrschenden Motilitätsphase in den Darm ausgestoßen. Dadurch tritt ein Verteilungseffekt ein und der Wirkstoff kann an größeren Bereichen der Darmschleimhaut seine Wirkung entfalten als eine Einzeldosisform.

Es wurde überraschenderweise gefunden, daß Budesonid-Pellets mit kontrolliertem Freigabeprofil erhalten werden können, wenn der Wirkstoff von einer freigabesteuernden Membran aus zwei separaten Lackschichten, einer Lackschicht I aus 80 bis 97% magensaftunlöslichen, darmsaftlöslichen Lacken und 3 bis 20% magen- und darmsaftunlöslichen Lacken, und einer Lackschicht II aus 100% magen- und darmsaftunlöslichen Lacken, umhüllt ist.

Gegenstand der Erfindung sind somit Budesonid-Pellets mit kontrolliertem Freigabeprofil, die dadurch gekennzeichnet sind, daß sie von innen nach außen

a) Neutralpellets
b) eine Wirkstoffschicht aus mikronisiertem Budesonid und einem oder mehreren wasserlöslichen Hilfsstoffen,
c) eine Lackschicht I aus 80 bis 97% magensaftunlöslichen, darmsaftlöslichen Lacken und 3 bis 20% magen- und darmsaftunlöslichen Lacken in einer Menge von 4-16 Gew.-%, bezogen auf die lackierten Wirkstoffpellets (bevorzugt 8-12%), und
d) eine Lackschicht II aus 100% magen- und darmsaftunlöslichen Lacken,

enthalten.

Gegenstand der Erfindung ist weiterhin ein Verfahren zur Herstellung von Budesonid-Pellets mit kontrolliertem Freigabeprofil, das dadurch gekennzeichnet ist, daß man mikronisiertes Budesonid als Wirkstoff in einem Lösungsmittelgemisch aus Alkohol:Wasser von 0:100 bis 20:80 unter Zugabe eines oder mehrerer wasserlöslicher Hilfsstoffe suspendiert, die Suspension auf Neutralpellets aufsprüht, nach Trocknen eine Lacksuspension I aus 80 bis 97% magensaftunlöslichen, darmsaftlöslichen Lacken und 3 bis 20% magen- und darmsaftunlöslichen Lacken aufsprüht, und nach Trocknen eine Lacksuspension II aus 100% magen- und darmsaftunlöslichen Lacken aufsprüht und die Wirkstoffpellets trocknet.

Bevorzugt enthalten die Pellets 75-95 Gew.-% Neutralpellets, 0,4-20 Gew.-% der Wirkstoffschicht, 4-16 Gew.-% (bevorzugter 8-12 Gew.-%) der Lackschicht I und 0,1-3 Gew.-% (bevorzugter 0,5-1 Gew.-%) der Lackschicht II jeweils bezogen auf lackierte Wirkstoffpellets.

Bevorzugt enthält die Lackschicht I Eudragit L, Eudragit S, Eudragit RS und Eudragit RL, die Lackschicht II Eudragit RL und Eudragit RS. Dabei beträgt in der Lackschicht I der Gehalt an Eudragit L 20 bis 77%, bevorzugt 40 bis 50%, an Eudragit S 20 bis 77%, bevorzugt 40 bis 50%, an Eudragit RS, Eudragit RL 3 bis 20%, bevorzugt 6 bis 12%, wobei die Summe aus Eudragit L und Eudragit S 80 bis 97% beträgt. Der Gehalt an Eudragit RL und Eudragit RS in der Lackmembran II beträgt 100%, wobei der Anteil an RL 0-100% und des RS 100-0% beträgt.

Der Gehalt an mikronisiertem Budesonid pro Dosiseinheit beträgt 0,5-20 mg, bevorzugt 0,5-5 mg, bevorzugter 0,6-3 mg. Dies entspricht 0,1-20 Gew.-%, bevorzugt 0,1-5 Gew.-%, bevorzugter 0,2-3 Gew.-% Budesonid, bezogen auf die lackierten Wirkstoffpellets. Dabei enthält eine Dosiseinheit ca. 150-750 einzelne Pellets. Die Teilchengröße des mikronisierten Budesonids beträgt 5 bis 25 $\mu$m, bevorzugt ca. 10 Mikrometer ($\mu$m).

Um hohe lokale Wirkstoffkonzentrationen und die Ausbreitung des Wirkstoffes über einen möglichst weiten entzündeten Darmbereich zu erzielen, wurde die Wirkstoffdosis auf hunderte von selbstständigen freigabesteuernden ca. 1 mm großen Arzneiformen (Pellets) verteilt (Pelletsverteilungseffekt) Bevorzugt ist die Wirkstoffmenge auf etwa 250 einzelne Pellets verteilt. Dabei enthält ein Pellet 0,003 mg bis 0,1 mg, bevorzugt 0,004 mg Budesonid. Die Pellets werden geeigneterweise zu Einzeldosiseinheiten zusammengefaßt. Dazu können sie von jeder beliebigen pharmazeutisch verträglichen Umhüllung umgeben sein. Die Pellets können dabei in Form von Kapseln, Tabletten, als Granulat oder in Form von Briefchen vorliegen, die in herkömmlicher Weise formuliert worden sind. Ferner können die Arzneimittel Formulierungshilfsmittel wie Suspendierungs-, Stabilisierungs- und/oder Dispergierungsmittel enthalten.

Bei Eudragit L und S handelt es sich um Acryl-/Methacryl-säure-Copolymere mit Carboxylgruppen, wodurch diese Lacke über pH 5,8 bzw. 6,8 löslich werden.

Genauer ist Eudragit L eine Poly(methacrylsäure, methylmethacrylat) mit einem Molverhältnis von 1:1 und einem Molekulargewicht von 135.000 oder eine Poly(ethylacrylat, methacrylsäure) mit einem Molverhältnis der monomeren Einheiten von 1:1 und einem Molekulargewicht von 250.000. Eudragit S ist ein Poly(methacrylsäure, methylmethacrylat) mit einem Molverhältnis der monomeren Einheiten von 1:2 und einem Molekulargewicht von 135.000.

Eudragit RS und RL sind Acryl-/Methacrylsäure-Copolymere mit quaternären Ammoniumgruppen. Sie sind im pH-Bereich 1 bis 7 unlöslich aber quellbar und unterliegen einer abgestuften Erosion.

Genauer ist Eudragit RL ein Poly(ethylacrylat, methylmethacrylat, trimethylamonioethylmethacrylatchlorid) mit einem Molverhältnis der monomeren Einheiten von 1:2:0,2 und einem Molekulargewicht von 150.000. Eudragit RS ist ein Poly(ethylacrylat, methylmethacrylat, trimethylamonioethylmethacrylatchlorid) mit einem Molverhältnis von 1:2:0,1 und einem Molekulargewicht von 150.000.

Zur Herstellung von Wirkstoffpellets rührt man normalerweise den Wirkstoff mit einem Klebstoff in einer alkoholischen Flüssigkeit an und sprüht diese Suspension bzw. Lösung auf sogenannte Starterpellets auf. Diese Starterpellets können beliebige pharmazeutisch verwendbare Neutralpellets wie Zucker/Maisstärkekügelchen sein. Gegebenenfalls können weitere Hilfsstoffe und Formulierungshilfsmittel, die einem Fachmann bekannt sind, verwendet werden. Die Überprüfung der Wirkstofffreigabe ergab jedoch völlig unbefriedigende Wirkstofffreisetzungen. Selbst nach 4 Stunden im künstlichen Darmsaft war noch nicht 80% des Wirkstoffes gelöst (siehe Vergleichsbeispiel 1) . Offensichtlich wird der Wirkstoff durch die genannten Lösungsmittel angelöst und bildet auf den Pellets eine zusammenhängende schwerlösliche Schicht. Dieses Verhalten ist völlig ungewöhnlich, da nur 3 mg Wirkstoff auf 300 mg Starterpellets (1%) aufgetragen wurde.

Auch Versuche mit Wasser, in dem sich der Wirkstoff praktisch nicht löste, führten zu unbefriedigenden Freisetzungen (Vergleichsbeispiel 2). Überraschenderweise konnte die gewünschte rasche Wirkstofffreisetzung dadurch gefunden werden, daß man den Wirkstoff in einem Lösungsmittelgemisch von Alkohol:Wasser von 0:100 bis 20:80 suspendiert und mindestens auf einen Teil Wirkstoff 2 Teile eines wasserlöslichen Hilfsstoffes zufügt. Besonders vorteilhaft hat sich hier das Verhältnis 1 Teil Budesonid:4 Teile $\alpha$-Lactose-Monohydrat erwiesen. Auch andere Hilfsstoffe wie Saccharose, Sorbit, Mannit, Mononatriumcitrat usw. sind geeignet (Beispiel 1).

Aus wirtschaftlichen Überlegungen sollte eine freigabesteuernde Membran auf den Pellets nicht mehr als 20% des Pelletgewichtes ausmachen.

Untersuchungen mit den üblichen magensaftresistenten Lacken wie Celluloseacetatphthalat, Hydroxypropylmethylcellulosephthalat und Eudragit L zeigten jedoch, daß bei der Prüfung der Wirkstofffreigabe bei pH 6,8 eine zu rasche Freisetzung selbst bei 20% Lack eintritt. Infolge der großen Oberfläche der Pellets erfolgt die Lackablösung so rasch, daß die aus den klinischen Untersuchungen begründete Freisetzung von

| 0 bis 2 Stunden | pH 1,2 | 0% Wirkstofffreigabe |
|---|---|---|
| 15 min | pH 6,8 | max. 5% Wirkstofffreigabe |
| 120 min | pH 6,8 | min. 90% Wirkstofffreigabe |

bei weitem nicht erreichbar war. In allen Fällen betrug die Freisetzung nach 15 min bei pH 6,8 bereits mehr als 50%. Außerdem war die Lackmenge unbefriedigend hoch (20%).

Auch die Kombination des Eudragit L mit dem sich erst bei pH 7,0 lösenden Eudragit S ergab unbefriedigende Ergebnisse. Selbst bei dem Verhältnis Eudragit S:Eudragit L von 8:2 konnte die Forderung (max. 5% nach 15 min) nicht erreicht werden (Vergleichsbeispiel 3).

Durch den Zusatz einer dritten Komponente, dem Eudragit RS, tritt bei wirtschaftlich vertretbaren Lackmengen (ca. 10 bis 15%) eine deutliche Reduzierung der Wirkstofffreigabe bei pH 6,8 ein. Bei pH 6,8 wird bei einer Lackmenge von 13,5% (Eudragit L:Eudragit S:Eudragit RS 46,5:46,5:7) nur noch 5 bis 10% innerhalb der ersten 15 min freigesetzt. Erhöht man den Eudragit RS-Anteil auf 12%, so erreicht man zwar die Reduzierung der Freigabe unter 5% nach 15 min, jedoch die Lackablösung verringert sich überraschenderweise so stark, daß nach 120 min erst ca. 70% freigesetzt sind (Beispiele 2 und 3). Es ist also offensichtlich, daß jede Wirkstofffreigabereduzierung in den ersten 15 min den Charakter der Lackmembran in der Weise verändert, daß die biologisch wichtige Freisetzungsforderung von mind. 90% nach 120 min nicht mehr erreichbar ist. Das aus medizinischen Gründen notwendige obige Freigabeprofil kann überraschenderweise durch eine separate zweite Lackmembran realisiert werden.

Dazu erhalten die nach Beispiel 4 hergestellten Pellets eine zweite Lackmembran aus Eudragit RL. Die Menge beträgt 0,1-3% (bezogen auf die lackierten Wirkstoffpellets). Wie das Beispiel 4 beweist, beträgt die Wirkstofffreigabe nach 15 min 0%, nach 60 min aber bereits 81% und nach 120 min 96%. Die zweite Lackmembran ist erfindungswesentlich, da sie die Freigabe nach 15 min auf 0% drückt, ohne die Freigabe nach 120 min wesentlich zu reduzieren. Dieses komplizierte Freigabeprofil kann man mit einer der Mischungen gemäß den Vergleichsbeispielen nicht erreichen. Die zweite Lackmembran besteht aus Eudragit RL oder RS bzw. aus Mischungen beider Lacke.

Die genannten Lacke werden wie üblich in Alkoholen wie Ethanol, Isopropanol und in Aceton gelöst. In der Regel werden ca. 10% Weichmacher (auf Lacktrockensubstanz bezogen) wie Triacetin oder Dibutylphthalat und Talk den Lacklösungen zugesetzt. Die Lacksuspensionen versprüht man in der Regel mit Zweistoffdüsen in geeigneten Coatinggeräten.

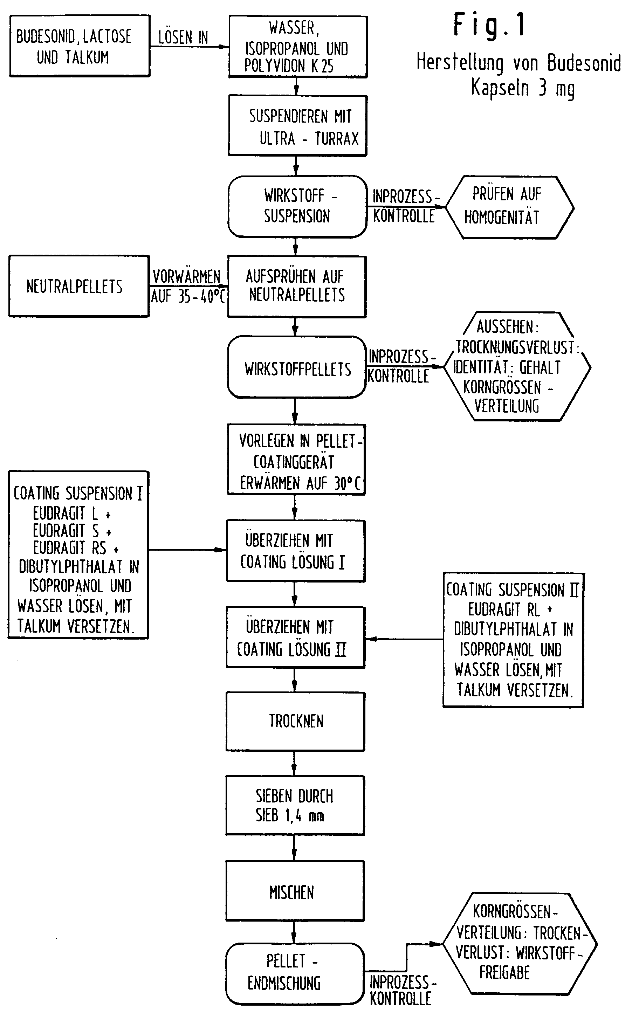

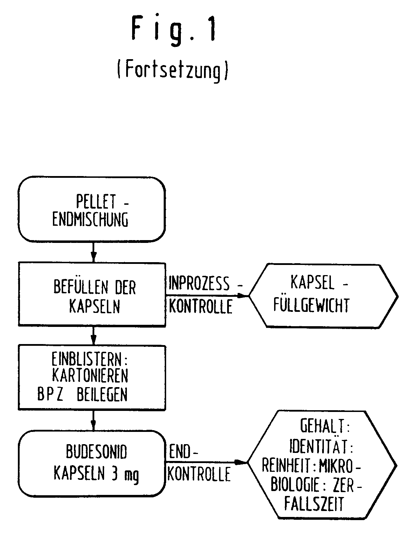

Der Herstellungsablauf ist in dem in Figur 1 gezeigten Ablaufsschema dargestellt. Im Prinzip handelt es sich um das Aufziehen des Wirkstoffs auf inerte Starterpellets, das Überziehen der Pellets mit pH-abhängig löslichen bzw. unlöslichen Lacken und das Verkapseln der Pellets.

Bei der Herstellung der Wirkstoffpellets muß von Charge zu Charge mit Gehaltsabweichungen in der Größenordnung 5 bis 10% gerechnet werden. Diese Abweichungen machen es erforderlich, den Wirkstoffgehalt der Pellets vor dem Kapsulieren zu bestimmen und entsprechend bei der Füllung der Kapseln zu berücksichtigen. Das Kapselfüllgewicht eines Ansatzes errechnet sich aus dem Ergebnis des Budesonid-Gehaltsbestimmungen jeder Pellet-Charge. Mit der entsprechenden Menge Neutralkügelchen wird das Gewicht erforderlichenfalls eingestellt.

Die Erfindung wird anhand der nachstehenden Beispiele näher erläutert

Vergleichsbeispiel 1

Die Wirkstofffreisetzung erfolgt unter nachfolgenden Bedingungen:

Paddle-Methode (USP XX II)
75 UpM, 37 +/-0,5 Grad Celsius

Medien:

pH 1,2:   2 g Natriumchlorid und 6,4 g HCl 37%ig pro 1000 ml Wasser
pH 6,8:   28 g Dinatriumhydrogenphosphat, wasserfrei und 4,34 g Zitronensäure pro 1000 ml Wasser; auf pH 6,8 eingestellt. In 1000 ml des Puffers werden 1,0 g Myrj 53 gelöst.

Zusammensetzung der Pellets:

| | |
|---|---|
| Neutralpellets (1) | 10,0 kg |
| Plasdone K 25 (2) | 0,1 kg |
| Talk (3) | 0,4 kg |
| Budesonid, mikron. (4) | 0,1 kg |
| | 10,6 kg |

Die Komponente 2 wird in 8 kg Ethanol/Wasser 8:2 gelöst und die Komponenten 3 und 4 mit einem Ultra-Turrax suspendiert. Die Suspension wird in einem Kessel mittels einer Zweistoffdüse auf die rotierenden Neutralpellets aufgesprüht.

Wirkstofffreigabe:

| | pH 1,2 | pH 6,8 |
|---|---|---|
| 15 min | 24% | 18% |
| 30 min | 45% | 43% |
| 60 min | 59% | 61% |
| 120 min | 67% | 69% |
| 180 min | 74% | 73% |

Vergleichsbeispiel 2

Gemäß Vergleichsbeispiel 1 werden Budesonid-Wirkstoffpellets hergestellt mit dem Unterschied, daß der Wirkstoff und das Talk in einer wässrigen Plasdone-Lösung (ca. 8 kg) suspendiert und auf die Neutralpellets aufgesprüht wird.

Wirkstofffreigabe:

| | pH 6,8 |
|---|---|
| 15 min | 34% |
| 30 min | 48% |
| 60 min | 58% |
| 120 min | 65% |
| 180 min | 69% |

Ergebnis:

Die Vergleichsbeispiele 1 und 2 zeigen, daß die medizinisch notwendige Freigabe bei pH 6,8 von mind. 90% nach 120 min mit diesen Pellets nicht realisierbar ist.

Beispiel 1

| Neutralpellets (1) | 10,0 kg |
| Plasdone K 25 (2) | 0,1 kg |
| Talk (3) | 0,4 kg |
| $\alpha$-Lactosemonohydrat (4) | 0,4 kg |
| Budesonid, mikronisiert (5) | 0,1 kg |

Die Komponente 2 wird in 3,3 kg Wasser/Ethanol 9:1 gelöst und die Komponenten 3 bis 5 mit dem Ultra-Turrax suspendiert. In einem rotierenden Kessel sprüht man diese Suspension auf die Neutralpellets mittels einer Zweistoffdüse.

Wirkstofffreigabe:

|  | pH 1,2 | pH 6,8 |
| --- | --- | --- |
| 15 min | 89,6% | 88,7% |
| 30 min | 98,1% | 98,4% |
| 60 min | 99,3% | 98,8% |
| 120 min | 99,5% | 99,1% |

Ergebnis:

Die Wirkstoffpellets geben den Wirkstoff unabhängig vom pH-Wert optimal rasch frei.

Vergleichsbeispiel 3

Wirkstoffpellets nach Beispiel 1 werden wie folgt besprüht:

| Wirkstoffpellets (1) | 5,50 kg |
| Eudragit S (2) | 0,97 kg |
| Eudragit L (3) | 0,24 kg |
| Dibutylphthalat (4) | 0,12 kg |
| Talk (5) | 1,00 kg |

Die Komponenten 2, 3, 4 werden in 15 kg Isopropanol/Wasser 95:5 gelöst und Talk einsuspendiert.
In einem rotierenden Kessel wird diese Lacksuspension auf 5,5 kg Wirkstoffpellets aufgesprüht (Lackmenge 22%, bezogen auf Starterpellets).

Wirkstofffreigabe:

| pH 1,2 | | pH 6,8 | |
|---|---|---|---|
| 60 min | 0% | 15 min | 42% |
| 120 min | 0% | 30 min | 83% |
| | | 60 min | 96% |

Die Pellets werden zuerst 120 min bei pH 1,2 gerührt und dann über ein Sieb quantitativ in die Pufferlösung pH 6,8 überführt.

Ergebnis:

Mit wirtschaftlich vertretbaren Lackmengen ist die gewünschte Freigabe von max. 5% nach 15 min bei pH 6,8 nicht realisierbar (Lackkombination Eudragit S:Eudragit L 8:2).

Beispiel 2

Exakt nach Vergleichsbeispiel 3 werden 5,5 kg Wirkstoffpellets nach Beispiel 1 mit folgender Lackkombination besprüht:

| Eudragit S | 0,345 kg |
|---|---|
| Eudragit L | 0,345 kg |
| Eudragit RS | 0,052 kg |
| Dibutylphthalat | 0,070 kg |
| Talk | 0,075 kg |

Die Lackkomponenten (Eudragit S:Eudragit L:Eudragit RS 46,5:46,5:7) löst man in 8,5 kg Isopropanol/Wasser 9:1 und verfährt weiter nach Vergleichsbeispiel 3.

Wirkstofffreigabe:

| pH 1,2 | | pH 6,8 | |
|---|---|---|---|
| 60 min | 0% | 15 min | 9,5% |
| 120 min | 0% | 30 min | 43,0% |
| | | 60 min | 75,0% |
| | | 120 min | 96,5% |

Ergebnis:

Die Wirkstofffreigabe nach 15 min bei pH 6,8 ist gegenüber Vergleichsbeispiel 3 deutlich gesenkt, erreicht jedoch nicht den notwendigen Wert von max. 5% nach 15 min bei pH 6,8. Die Freisetzung nach 120 min liegt über der Toleranz (mind. 90% nach 120 min).

Beispiel 3

Exakt nach Beispiel 2 werden Wirkstoffpellets nach Beispiel 1 mit nachfolgender Lackkombination besprüht:

| Eudragit S | 0,327 kg |
|---|---|
| Eudragit L | 0,327 kg |
| Eudragit RS | 0,089 kg |
| Dibutylphthalat | 0,070 kg |
| Talk | 0,075 kg |

Die Lackkomponenten (Eudragit S:Eudragit L:Eudragit RS 44:44:12) löst man in 8,5 kg Isopropanol/Wasser 9:1 und verfährt weiter nach Beispiel 2.

Wirkstofffreigabe:

| pH 1,2 | | pH 6,8 | |
|---|---|---|---|
| 60 min | 0% | 15 min | 2,4% |
| 120 min | 0% | 30 min | 26,4% |
| | | 60 min | 56,3% |
| | | 120 min | 78,1% |
| | | 180 min | 89,8% |

Ergebnis:

Die Erhöhung des Eudragit RS-Anteils auf 12% drückt die Freisetzung unter 5% nach 15 min bei pH 6,8, die Freisetzungsanforderung nach 120 min (mind. 90%) wird jedoch deutlich verfehlt.

Beispiel 4

Exakt nach Beispiel 3 werden 5,5 kg Wirkstoffpellets nach Beispiel 1 mit nachfolgenden Lackschichten besprüht:

Lackschicht I

| Eudragit S | 0,304 kg |
|---|---|
| Eudragit L | 0,304 kg |
| Eudragit RS | 0,052 kg |
| Dibutylphthalat | 0,065 kg |
| Talk | 0,065 kg |

Die Lackkombination (Eudragit S:Eudragit L:Eudragit RS 46:46:8) löst man in 7,6 kg Isopropanol/Wasser 9:1 und verfährt nach Vergleichsbeispiel 3. Die Lackmenge beträgt 12%.

Wirkstofffreigabe:

| pH 1,2 | | pH 6,8 | |
|---|---|---|---|
| 60 min | 0% | 15 min | 14,4% |
| 120 min | 0% | 30 min | 49,0% |
| | | 60 min | 84,0% |
| | | 120 min | 98,4% |

Lackschicht II

| Eudragit RL | 0,060 kg |
|---|---|
| Dibutylphthalat | 0,070 kg |
| Talk | 0,100 kg |

Eudragit RL löst man in 3,66 kg Isopropanol/Wasser 9:1 und fügt den Weichmacher und Talk mittels Ultra-Turrax zu. Die Lacksuspension sprüht man in einem Kessel auf die mit der Lackschicht I versehenen Pellets.

Wirkstofffreigabe:

| pH 1,2 | | pH 6,8 | |
|---|---|---|---|
| 60 min | 0% | 15 min | 0% |
| 120 min | 0% | 30 min | 28% |
| | | 60 min | 81% |
| | | 120 min | 97% |

Ergebnis:

Die zweite Lackschicht (0,92% Eudragit RL bezogen auf lackierte Wirkstoffpellets) reduziert wie gefordert die Freigabe nach 15 min bei pH 6,8 auf 0% ohne die Freisetzungsanforderung (120 min mind. 90%) zu verfehlen. Die Kombination zweier Lackschichten erweist sich zur Freigabesteuerung wesentlich besser als Lackkombinationen der erwähnten Lacke.

Beispiel 5

Es werden nach Beispiel 1 Wirkstoffpellets mit der dreifachen Wirkstoffmenge hergestellt.

| Neutralpellets | 10,0 kg |
|---|---|
| Plasdone | 0,1 kg |
| Talk | 0,4 kg |
| $\alpha$-Lactosemonohydrat | 1,2 kg |

(fortgesetzt)

| Budesonid, mikron. | 0,3 kg |
|---|---|
| | 12,0 kg |

Diese Pellets werden wie beschrieben mit nachfolgenden Lackschichten besprüht:

Lackschicht I

| Eudragit S | 0,49 kg |
|---|---|
| Eudragit L | 0,49 kg |
| Eudragit RL | 0,22 kg |
| Dibutylphthalat | 0,12 kg |
| Talk | 0,18 kg |

Lackschicht II

| Eudragit RS | 0,081 kg |
|---|---|
| Dibutylphthalat | 0,01 kg |
| Talk | 0,01 kg |

Wirkstofffreigabe:

| pH 1,2 | | pH 6,8 | |
|---|---|---|---|
| 60 min | 0% | 15 min | 1,8% |
| 120 min | 0% | 60 min | 75,3% |
| | | 120 min | 92,9% |

Ergebnis:

Mit der Lackkombination (Lackschicht I) Eudragit S:Eudragit L:Eudragit RL 41:41:18 (Lackmenge 10%) und der Lackschicht II mit 0,59% Eudragit RS erhält man ebenfalls das medizinisch notwendige Freigabeprofil.

Beispiel 6

Budesonid-Kapseln 3 mg

Zusammensetzung

|  |  |  | mg/Kapsel | Funktion |
|---|---|---|---|---|
| 1) | | Budesonid, mikr. | 3,0 | Wirkstoff |
| 2) | | Neutralpellets 1,0-1,18 mm | 300,0 | Starterkügelchen |
| 3) | | Lactose-Monohydrat | 12,0 | Füllstoff |
| 4) | | Plasdone K 25 mittleres MG 25000 | 0,9 | Bindemittel |
| 5) | | Eudragit L | 18,3 | Lack |
| 6) | | Eudragit S | 18,3 | Lack |
| 7) | | Eudragit RS | 3,0 | Lack |
| 8) | | Eudragit RL | 2,1 | Lack |
| 9) | | Dibutylphthalat | 4,2 | Weichmacher |
| 10) | | Talk | 44,7 | Trennmittel |
| | | Kapselinhalt | 406,5 | |
| 11) | | Hartgelatine-Kapsel Gr. I | 77,0 | |
| 12) | | Wasser*, gereinigt | | ca. 69 mg |
| 13) | | Isopropanol* | | ca. 409 mg |
| | | Kapsel-Gesamtgewicht | 483,5 | |

*) flüchtige Bestandteile

Beispiel 7

| | |
|---|---|
| Lackierte Wirkstoffpellets nach Beispiel 6 (1) | 1,35 kg |
| Mikrokristalline Cellulose (2) | 4,00 kg |
| Lactose, direkt tablettierbar (3) | 1,60 kg |
| Siliciumdioxid (4) | 0,10 kg |
| Magnesiumstearat (5) | 0,10 kg |
| | 7,15 kg |

Die Komponenten (2) bis (4) werden durch ein Sieb mit einer Maschenweite von 1,0 mm gesiebt, dann wird die Komponente (1) hinzugefügt und es wird 10 Minuten lang vermischt. Anschließend wird die Komponente (5) hinzugeben und es wird nochmals 5 Minuten lang gemischt. Das Gemisch wird zu Tabletten mit einem Durchmesser von 13 mm verpreßt. Das Gewicht der Tablette beträgt 715 mg, der Gehalt an Budesonid 1 mg. Die Tablette zerfällt nach 2 Minuten unter Freisetzung der lackierten Budesonidwirkstoffpellets.

Beispiel 8

| | |
|---|---|
| Lackierte Wirkstoffpellets nach Beispiel 6 (1) | 8,13 kg |
| Sorbit Instant-Granulat (2) | 40,00 kg |
| Natriumcarboxymethylcellulose (3) | 2,15 kg |
| Zitronensäure (4) | 0,90 kg |
| Zitronenaroma (5) | 0,82 kg |
| | 52,00 kg |

Die Komponenten (2) bis (4) werden durch ein Sieb mit einer Maschenweite von 1,0 mm gesiebt und zusammen mit der Komponente (1) 15 Minuten lang vermischt. Das Gemisch wird in Beuteln zu je 2,6 g abgefüllt.

Vor Gebrauch wird ein Beutel in einem Glas Wasser unter Rühren suspendiert. Es entsteht ein gut schmeckendes Getränk, bei dem die Pellets infolge der Viskositätserhöhung kaum sedimentieren. Ein Beutel enthält 406,5 mg Pellets, was 3 mg Budesonid entspricht.

Beispiel 9

Es wurden 375.000 Hartgelatinekapseln mit den erfindungsgemäßen Budesonid-Pellets zu je 3 mg Budesonid hergestellt. Das Freigabeprofil wurde gemäß Vergleichsbeispiel 1 ermittelt. Die folgenden Werte wurden erhalten:

| | | |
|---|---|---|
| 2h | pH 1,2 | 0% |
| 15 min | pH 6,8 | 0% |
| 30 min | pH 6,8 | ~55% |
| 60 min | pH 6,8 | >90% |

**Patentansprüche**

1. Budesonid-Pellets mit kontrolliertem Freigabeprofil, wobei die Wirkstofffreigabe nach 15 min. 0 % beträgt und wobei mindestens 90 % des Wirkstoffes nach 120 min. freigesetzt werden, dadurch **gekennzeichnet**, daß sie von innen nach außen

   a) Neutralpellets
   b) eine Wirkstoffschicht aus mikronisiertem Budesonid und einem oder mehreren wasserlöslichen Hilfsstoffen,
   c) eine Lackschicht I aus 80 bis 97% magensaftunlöslichen, darmsaftlöslichen Lacken und 3 bis 20% magen- und darmsaftunlöslichen Lacken, und
   d) eine Lackschicht II aus 100% magen- und darmsaftunlöslichen Lacken,

   enthalten.

2. Budesonid-Pellets mit kontrolliertem Freigabeprofil nach Anspruch 1, dadurch **gekennzeichnet**, daß sie von innen nach außen

   a) 75 bis 95 Gew.-% Neutralpellets,
   b) 0,4 bis 20 Gew.-% einer Wirkstoffschicht aus mikronisiertem Budesonid und einem oder mehreren wasserlöslichen Hilfsstoffen,
   c) 4 bis 16 Gew.-% einer Lackschicht I aus 80 bis 97% magensaftunlöslichen, darmsaftlöslichen Lacken und 3 bis 20% magen- und darmsaftunlöslichen Lacken, und
   d) 0,1 bis 3 Gew.-% einer Lackschicht II aus 100% magen- und darmsaftunlöslichen Lacken, Jeweils bezogen auf lackierte Wirkstoffpellets,

enthalten.

3. Pellets nach Anspruch 1 oder 2, dadurch **gekennzeichnet**, daß die Lackschicht I Eudragit® L, Eudragit® S, Eudragit® RS und Eudragit® RL enthält.

4. Pellets nach mindestens einem der Ansprüche 1 bis 3, dadurch **gekennzeichnet**, daß die Lackschicht II Eudragit® RL und Eudragit® RS enthält.

5. Pellets nach mindestens einem der Ansprüche 1 bis 4, dadurch **gekennzeichnet**, daß die Lackschicht I 20 bis 77% Eudragit® L, 20 bis 77% Eudragit® S, 3 bis 20% Eudragit® RS und Eudragit® RL enthält, wobei die Summe aus Eudragit® L und Eudragit® S 80 bis 97%, beträgt.

6. Pellets nach mindestens einem der Ansprüche 1 bis 5, dadurch **gekennzeichnet**, daß die Lackschicht II 0-100% Eudragit® RL und 100-0 % Eudragit® RS, enthält.

7. Pellets nach mindestens einem der Ansprüche 1 bis 6, dadurch **gekennzeichnet**, daß der wasserlösliche Hilfsstoff aus der Gruppe $\alpha$-Lactose-Monohydrat, Saccharose, Sorbic, Mannit, Mononatriumcitrat ausgewählt ist.

8. Pellets nach mindestens einem der Ansprüche 1 bis 7, dadurch **gekennzeichnet**, daß der Gehalt an Budesonid 0,1-5 Gew.-%, bezogen auf lackierte Wirkstoffpellets, beträgt.

9. Pellets nach mindestens einem der Ansprüche 1 bis 8, dadurch **gekennzeichnet**, daß die Teilchengröße des mikronisierten Budesonids im Bereich von 5 bis 25 $\mu$m liegt.

10. Pellets nach mindestens einem der Ansprüche 1 bis 9, dadurch **gekennzeichnet**, daß sie in Form von Kapseln, Tabletten oder als Granulat vorliegen.

11. Verfahren zur Herstellung von Budesonid-Pellets mit kontrolliertem Freigabeprofil nach mindestens einem der Ansprüche 1 bis 10, dadurch **gekennzeichnet**, daß man mikronisiertes Budesonid als Wirkstoff in einem Lösungsmittelgemisch aus Alkohol:Wasser von 0:100 bis 20:80 unter Zugabe eines oder mehrerer wasserlöslicher Hilfsstoffe suspendiert, die Suspension auf Neutralpellets aufsprüht, nach Trocknen eine Lacksuspension I aus 80 bis 97% magensaftunlöslichen, darmsaftlöslichen Lacken und 3 bis 20% magen- und darmsaftunlöslichen Lacken aufsprüht, und nach Trocknen eine Lacksuspension II aus 100% magen- und darmsaftunlöslichen Lacken aufsprüht und die Wirkstoffpellets trocknet.

12. Verfahren nach Anspruch 11, dadurch **gekennzeichnet**, daß man
bis 0,1-5 Gew.-% mikronisiertes Budesonid als Wirkstoff in einem Lösungsmittelgemisch aus Alkohol:Wasser von 0:100 bis 20:80 unter Zugabe eines oder mehrerer wasserlöslicher Hilfsstoffe suspendiert,
die Suspension auf Neutralpellets aufsprüht,
nach dem Trocknen 4 bis 16 Gew.-% einer Lacksuspension I aus 80 bis 97% magensaftunlöslichen, darmsaftlöslichen Lakken und 3 bis 20% magen- und darmsaftunlöslichen Lacken, bezogen auf lackierte Wirkstoffpellets, aufsprüht, und
nach dem Trocknen 0,1 bis 3 Gew.-% einer Lacksuspension II aus 100% magen- und darmsaftunlöslichen Lacken, bezogen auf lackierte Wirkstoffpellets, aufsprüht und die Wirkstoffpellets trocknet.

13. Verfahren nach Anspruch 11 oder 12, dadurch **gekennzeichnet**, daß die Lacksuspension I Eudragit® L, Eudragit® S, Eudragit® RS und Eudragit® RL enthält.

14. Verfahren nach mindestens einem der Ansprüche 11 bis 13, dadurch **gekennzeichnet**, daß die Lacksuspension II Eudragit® RL und Eudragit® RS enthält.

15. Verfahren nach mindestens einem der Ansprüche 11 bis 14, dadurch **gekennzeichnet**, daß die Lacksuspension I 20 bis 77% Eudragit® L, 20 bis 77% Eudragit® S, 3 bis 20% Eudragit® RS und Eudragit® RL enthält, wobei die Summe aus Eudragit® L und Eudragit® S 80 bis 97% beträgt.

16. Verfahren nach mindestens einem der Ansprüche 11 bis 15, dadurch **gekennzeichnet**, daß die Lacksuspension II 0-100% Eudragit® RL und 100-0% Eudragit® RS, enthält.

**17.** Verfahren nach mindestens einem der Ansprüche 11 bis 16, dadurch **gekennzeichnet**, daß der wasserlösliche Hilfsstoff aus der Gruppe α-Lactose-Monohydrat, Saccharose, Sorbit, Mannit, Mononatriumcitrat ausgewählt wird.

**18.** Verfahren nach mindestens einem der Ansprüche 11 bis 17, dadurch **gekennzeichnet**, daß 0,1 bis 5 Gew.-% Budesonid, bezogen auf lackierte Wirkstoffpellets, verwendet werden.

**19.** Verfahren nach mindestens einem der Ansprüche 11 bis 18, dadurch **gekennzeichnet**, daß mikronisiertes Budesonid mit einer Teilchengröße im Bereich von 5 bis 25 μm verwendet wird.

**20.** Verfahren nach mindestens einem der Ansprüch 11 bis 19, dadurch **gekennzeichnet**, daß man eine geeignete Menge Pellets in Hartgelatinekapseln oder, mit geeigneten Hilfsstoffen in Sachets abfüllt, bzw. mit geeigneten Hilfsstoffen zu Tabletten verpreßt.

## Claims

**1.** Budesonide pellets with controlled release profile, wherein the release of active substance after 15 min. is 0 %, and at least 90 % of active substance is released after 120 min., characterized in that they comprise, from the inside to the outside,

a) neutral pellets
b) an active substance layer comprising micronized budesonide and one or more water-soluble auxiliary agents,
c) a lacquer layer I comprising 80 to 97 % of lacquers which are insoluble in gastric juice and soluble in intestinal juice and 3 to 20 % of lacquers which are insoluble in gastric and intestinal juices, and
d) a lacquer layer II consisting of 100 % lacquers which are insoluble in gastric and intestinal juices.

**2.** Budesonide pellets with controlled release profile as claimed in claim 1, characterized in that they comprise, from the inside to the outside,

a) 75 to 95 % by weight of neutral pellets,
b) 0.4 to 20 % by weight of an active substance layer comprising micronized budesonide and one or more water-soluble auxiliary agents,
c) 4 to 16 % by weight of a lacquer layer I comprising 80 to 97 % of lacquers which are insoluble in gastric juice and soluble in intestinal juice and 3 to 20 % of lacquers which are insoluble in gastric and intestinal juices, and
d) 0.1 to 3 % by weight of a lacquer layer II consisting of 100 % lacquers which are insoluble in gastric and intestinal juices, in each case related to lacquered active substance pellets.

**3.** Pellets as claimed in claim 1 or 2, characterized in that lacquer layer I comprises Eudragit®L, Eudragit®S, Eudragit®RS and Eudragit®RL.

**4.** Pellets as claimed in at least one of claims 1 to 3, characterized in that lacquer layer II comprises Eudragit®RL and Eudragit®RS.

**5.** Pellets as claimed in at least one of claims 1 to 4, characterized in that lacquer layer I comprises 20 to 77 % of Eudragit®L, 20 to 77 % of Eudragit®S, 3 to 20 % of Eudragit®RS and Eudragit®RL, where the total of Eudragit®L and Eudragit®S is 80 to 97 %.

**6.** Pellets as claimed in at least one of claims 1 to 5, characterized in that lacquer layer II comprises 0-100 % Eudragit®RL and 100-0 % Eudragit®RS.

**7.** Pellets as claimed in at least one of claims 1 to 6, characterized in that the water-soluble auxiliary agent is selected from the group of α-lactose monohydrate, sucrose, sorbitol, mannitol, monosodium citrate.

**8.** Pellets as claimed in at least one of claims 1 to 7, characterized in that the budesonide content is 0.1-5 % by weight related to lacquered active substance pellets.

**9.** Pellets as claimed in at least one of claims 1 to 8, characterized in that the particle size of the micronized budesonide is in the range from 5 to 25 μm.

10. Pellets as claimed in at least one of claims 1 to 9, characterized in that they are in the form of capsules, tablets or granules.

11. A process for producing budesonide pellets with controlled release profile as claimed in at least one of claims 1 to 10, characterized in that micronized budesonide is suspended as active substance in a solvent mixture consisting of alcohol water from 0:100 to 20:80 with the addition of one or more water-soluble auxiliary agents, the suspension is sprayed onto neutral pellets, after drying a lacquer suspension I consisting of 80 to 97 % of lacquers which are insoluble in gastric juice and soluble in intestinal juice and 3 to 20 % of lacquers which are insoluble in gastric and intestinal juices is sprayed on, and after drying a lacquer suspension II consisting of 100 % lacquers which are insoluble in gastric and intestinal juices is sprayed on, and the active substance pellets are dried.

12. The process as claimed in claim 11, characterized in that
up to 0.1-5 % by weight of micronized budesonide is suspended as active substance in a solvent mixture consisting of alcohol water from 0:100 to 20:80 with the addition of one or more water-soluble auxiliary agents,
the suspension is sprayed onto neutral pellets,
after drying 4 to 16 % by weight, related to lacquered active substance pellets, of a lacquer suspension I consisting of 80 to 97 % of lacquers which are insoluble in gastric juice and soluble in intestinal juice and 3 to 20 % of lacquers which are insoluble in gastric and intestinal juices is sprayed on, and
after drying 0.1 to 3 % by weight, related to lacquered active substance pellets, of a lacquer suspension II consisting of 100 % lacquers which are insoluble in gastric and intestinal juices is sprayed on, and the active substance pellets are dried.

13. The process as claimed in claim 11 or 12, characterized in that lacquer suspension I comprises Eudragit$^{®}$L, Eudragit$^{®}$S, Eudragit$^{®}$RS and Eudragit$^{®}$RL.

14. The process as claimed in at least one of claims 11 to 13, characterized in that lacquer suspension II comprises Eudragit$^{®}$RL and Eudragit$^{®}$RS.

15. The process as claimed in at least one of claims 11 to 14, characterized in that lacquer suspension I comprises 20 to 77 % of Eudragit$^{®}$L, 20 to 77 % of Eudragit$^{®}$S, 3 to 20 % of Eudragit$^{®}$RS and Eudragit$^{®}$RL, where the total of Eudragit$^{®}$L and Eudragit$^{®}$S is 80 to 97 %.

16. The process as claimed in at least one of claims 11 to 15, characterized in that lacquer suspension II comprises 0-100 % Eudragit$^{®}$RL and 100-0 % Eudragit$^{®}$RS.

17. The process as claimed in at least one of claims 11 to 16, characterized in that the water-soluble auxiliary agent is selected from the group of $\alpha$-lactose monohydrate, sucrose, sorbitol, mannitol, monosodium citrate.

18. The process as claimed in at least one of claims 11 to 17, characterized in that 0.1 to 5 % by weight of budesonide is used, related to lacquered active substance pellets.

19. The process as claimed in at least one of claims 11 to 18, characterized in that micronized budesonide having a particle size in the range from 5 to 25 $\mu$m is used.

20. The process as claimed in at least one of claims 11 to 19, characterized in that a suitable amount of pellets is packed in hard gelatin capsules or, with suitable auxiliary agents, in sachets or compressed with suitable auxiliary agents to tablets.

**Revendications**

1. Granules de budésonide à profil de libération maîtrisé, où la libération du principe actif après 15 minutes est de 0% et où au moins 90% du principe actif sont libérés après 120 minutes, caractérisés en ce que les granules contiennent, de l'intérieur vers l'extérieur:

   a) des granules neutres
   b) une couche de principe actif qui se compose de budésonide micronisé et d'un ou plusieurs adjuvants solubles dans l'eau,
   c) une couche de laque I constituée de 80 à 97% de laques insolubles dans le suc gastrique mais solubles dans le suc intestinal et de 3 à 20% de laques insolubles dans le suc gastrique et dans le suc intestinal et

d) une couche de laque II constituée de 100% de laques insolubles dans le suc gastrique et le suc intestinal.

2. Granules de budésonide à profil de libération maîtrisé suivant la revendication 1, caractérisé en ce qu'ils contiennent, de l'intérieur vers l'extérieur,

a) 75 à 95% en poids de granules neutres,

b) 0,4 à 20% en poids d'une couche de principe actif de budésonide micronisé et d'un ou plusieurs adjuvants solubles dans l'eau,

c) 4 à 16% d'une couche de laque I constituée de 80 à 97% de laques insolubles dans le suc gastrique mais solubles dans le suc intestinal et de 3 à 20% de laques insolubles dans le suc gastrique et dans le suc intestinal et

d) 0,1 à 3% en poids d'une couche de laque II constituée de 100% de laques insolubles dans le suc gastrique et dans le suc intestinal, à chaque fois par rapport aux granules de principe actif laqués.

3. Granules suivant la revendication 1 ou 2, qui se caractérisent en ce que la couche de laque I contient les produits qui suivent : Eudragit$^®$ L, Eudragit$^®$ S, Eudragit$^®$ RS et Eudragit$^®$ RL.

4. Granules suivant au moins l'une des revendications 1 à 3, caractérisés en ce que la couche de laque II contient les produits qui suivent : Eudragit$^®$ RL et Eudragit$^®$ RS.

5. Granules suivant au moins l'une des revendications 1 à 4, caractérisés en ce que la couche de laque I contient 20 à 77% d'Eudragit$^®$ L, 20 à 77% d'Eudragit$^®$ S, 3 à 20% d'Eudragit$^®$ RS et d'Eudragit$^®$ RL, où la somme de l'Eudragit$^®$ L et de l'Eudragit$^®$ S représente de 80 à 97%.

6. Granules suivant au moins l'une des revendications 1 à 5, caractérisés en ce que la couche de laque II contient de 0 à 100% d'Eudragit$^®$ RL et 100 à 0% d'Eudragit$^®$ RS.

7. Granules suivant au moins l'une des revendications 1 à 6, caractérisés en ce que l'adjuvant soluble dans l'eau est choisi dans le groupe formé par l'$\alpha$-lactose monohydraté, le saccharose, la sorbite, la mannite et le citrate monosodique.

8. Granules suivant l'une des revendications 1 à 7, caractérisés en ce que la teneur en budésonide varie de 0,1 à 5% en poids, par rapport aux granules de principe actif laqués.

9. Granules suivant au moins l'une des revendications 1 à 8, caractérisés en ce que le calibre des particules du budésonide micronisé varie de 5 à 25 $\mu$m.

10. Granules suivant au moins l'une des revendications 1 à 9, caractérisés en ce qu'ils se présentent sous la forme de gélules ou capsules, de comprimés, ou sous la forme d'un granulé.

11. Procédé de fabrication de granules de budésonide à profil de libération régulé ou maîtrisé suivant l'une quelconque des revendications 1 à 10, caractérisé en ce que l'on met en suspension du budésonide micronisé servant de principe actif dans un mélange de solvants constitué d'alcool et d'eau de 0:100 à 20:80, en recourant à l'addition d'un ou plusieurs adjuvants solubles dans l'eau, on pulvérise la suspension sur des granules neutres, après le séchage, on pulvérise une suspension de laque I constituée de 80 à 97% de laques insolubles dans le suc gastrique, mais solubles dans le suc intestinal et de 3 à 20% de laques insolubles dans le suc gastrique et dans le suc intestinal et, après le séchage, on pulvérise une suspension de laque II constituée de 100% de laques insolubles dans le suc gastrique et dans le suc intestinal et on sèche les granules de principe actif.

12. Procédé suivant la revendication 11, caractérisé en ce que l'on met en suspension de 0,1 à 5% en poids de budésonide micronisé servant de principe actif dans un mélange de solvants constitué d'alcool et d'eau de 0:100 à 20:80, en recourant à l'addition d'un ou plusieurs adjuvants solubles dans l'eau,

on pulvérise la suspension sur des granules neutres,

après le séchage, on pulvérisé de 4 à 16% en poids d'une suspension de laque I constituée de 80 à 97% de laques insolubles dans le suc gastrique mais solubles dans le suc intestinal et de 3 à 20% en poids de laques insolubles dans le suc gastrique et dans le suc intestinal, par rapport aux granules de principe actif laqués et

après le séchage, on pulvérise de 0,1 à 3% en poids d'une suspension de laque II constituée de 100% de laques insolubles dans le suc gastrique et dans le suc intestinal, par rapport aux granules de principe actif laqués et on sèche les granules de principe actif.

13. Procédé suivant la revendication 11 ou 12, caractérisé en ce que la suspension de laque I contient les produits qui suivent : Eudragit<sup>®</sup> L, Eudragit<sup>®</sup> S, Eudragit<sup>®</sup> RS et Eudragit<sup>®</sup> RL.

14. Procédé suivant au moins l'une des revendications 11 à 13, caractérisé en ce que la suspension de laque II contient les produits qui suivent : Eudragit<sup>®</sup> RL et Eudragit<sup>®</sup> RS.

15. Procédé suivant au moins l'une des revendications 11 à 14, caractérisé en ce que la suspension de laque I contient 20 à 77% d'Eudragit<sup>®</sup> L, 20 à 77% d'Eudragit<sup>®</sup> S, 3 à 20% d'Eudragit<sup>®</sup> RS et d'Eudragit<sup>®</sup> RL, où la somme de l'Eudragit<sup>®</sup> L et de l'Eudragit<sup>®</sup> S représente de 80 à 97%.

16. Procédé suivant au moins l'une des revendications 11 à 15, caractérisé en ce que la suspension de laque II contient de 0 à 100% d'Eudragit<sup>®</sup> RL et 100 à 0% d'Eudragit<sup>®</sup> RS.

17. Procédé suivant au moins l'une des revendications 11 à 16, caractérisé en ce que l'adjuvant soluble dans l'eau est choisi dans le groupe formé par l'$\alpha$-lactose monohydraté, le saccharose, la sorbite, la mannite et le citrate monosodique.

18. Procédé suivant au moins l'une des revendications 11 à 17, caractérisé en ce que l'on utilise de 0,1 à 5% en poids de budésonide, par rapport aux granules de principe actif laqués.

19. Procédé suivant au moins l'une des revendications 11 à 18, caractérisé en ce que l'on utilise du budésonide micronisé d'un calibre des particules qui varie de 5 à 25 $\mu$m.

20. Procédé suivant au moins l'une des revendications 11 à 19, caractérisé en ce que l'on introduit une quantité appropriée de granules dans des gélules ou capsules en gélatine dure ou en ce que l'on introduit une quantité appropriée de granules dans des sachets avec des adjuvants convenables, ou en ce qu'on les comprime en comprimés avec des adjuvants convenables.

**Fig.1**

Herstellung von Budesonid
Kapseln 3 mg

| BUDESONID, LACTOSE UND TALKUM | LÖSEN IN → | WASSER, ISOPROPANOL UND POLYVIDON K 25 |
|---|---|---|

↓

SUSPENDIEREN MIT ULTRA - TURRAX

↓

WIRKSTOFF - SUSPENSION → INPROZESS-KONTROLLE → PRÜFEN AUF HOMOGENITÄT

| NEUTRALPELLETS | VORWÄRMEN AUF 35-40°C | AUFSPRÜHEN AUF NEUTRALPELLETS |
|---|---|---|

↓

WIRKSTOFFPELLETS → INPROZESS-KONTROLLE → AUSSEHEN: TROCKNUNGSVERLUST: IDENTITÄT: GEHALT KORNGROSSEN - VERTEILUNG

↓

VORLEGEN IN PELLET-COATINGGERÄT ERWÄRMEN AUF 30°C

COATING SUSPENSION I EUDRAGIT L + EUDRAGIT S + EUDRAGIT RS + DIBUTYLPHTHALAT IN ISOPROPANOL UND WASSER LÖSEN, MIT TALKUM VERSETZEN. →

↓

ÜBERZIEHEN MIT COATING LÖSUNG I

↓

ÜBERZIEHEN MIT COATING LÖSUNG II ← COATING SUSPENSION II EUDRAGIT RL + DIBUTYLPHTHALAT IN ISOPROPANOL UND WASSER LÖSEN, MIT TALKUM VERSETZEN.

↓

TROCKNEN

↓

SIEBEN DURCH SIEB 1,4 mm

↓

MISCHEN

↓

PELLET - ENDMISCHUNG → INPROZESS-KONTROLLE → KORNGRÖSSEN-VERTEILUNG: TROCKEN-VERLUST: WIRKSTOFF-FREIGABE

# Fig. 1

## (Fortsetzung)